(19)

Europäisches
Patentamt
European
Patent Office
Office européen
des brevets

(11) **EP 1 923 086 B1**

(12) **EUROPEAN PATENT SPECIFICATION**

(45) Date of publication and mention
of the grant of the patent:
**04.04.2012 Bulletin 2012/14**

(51) Int Cl.:
***A61M 5/34*** *(2006.01)*

(21) Application number: **07120460.6**

(22) Date of filing: **12.11.2007**

(54) **Luer-lock type cylindrical tip of syringe**

Zylindrische Luer-Lock-Spritzenspitze

Pointe de seringue cylindrique à verrouillage Luer

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **15.11.2006 JP 2006308817**

(43) Date of publication of application:
**21.05.2008 Bulletin 2008/21**

(73) Proprietor: **ARTE CORPORATION
Tokyo (JP)**

(72) Inventors:
• **Shimazaki, Seiji
Takahagi-shi, Ibaraki-Ken (JP)**

• **Takeshima, Yasuhiko
Tokyo (JP)**

(74) Representative: **Metman, Karel Johannes et al
De Vries & Metman
Overschiestraat 180
1062 XK Amsterdam (NL)**

(56) References cited:
**FR-A- 2 863 162      JP-A- 9 154 947
US-A- 2 755 801      US-A- 3 402 713
US-A- 3 491 757      US-A- 4 240 422
US-A- 4 927 417**

## Description

Description of the Related Art

The combined container-syringes are delivered to the pharmaceutical company in a state such that the injection needles are attached in advance to the luer-lock type cylindrical tips. The delivered combined container-syringes are sterilized at the pharmaceutical company. After sterilization, operations such as filling the syringes with the drug solution, assembly, inspection, and packaging are automatically and speedily performed by machine. The injection needles which have been attached to the cylindrical tips are loosened by mechanical vibration or load more easily than before delivery. Furthermore, in the above-described machine, there are many machines which are manufactured without consideration of the looseness of the injection needle. The syringes in which the drug solution has been filled are handled as above and shipped to the market or medical institutions through the medical wholesale or the like. Disposable syringes with needles in which the injection needles are attached in advance to the luer-lock type cylindrical tips are also shipped to the market, the medical institutions, and medical wholesale after the operations such as assembly, inspection, packaging, and sterilization. However, the injection needle becomes easily loosened and pulled out during these operations or during transport through the distribution channel. Accordingly, even after these processes or handling after the delivery, the injection needles which have been attached to the syringes should not become loosened or pulled out.

A luer-lock type cylindrical tip in which thickness of a base of a cylindrical tip which connects a cylindrical luer tip with a cylindrical luer-lock portion disposed outside the luer tip and having a lock screw is increased in size is known. With this luer-lock type cylindrical tip, at the time of fitting an inner hole of a needle base of an injection needle to the luer tip by screwing an outer peripheral screw of the needle base to a lock screw of the luer-lock portion, it is possible to prevent the luer-lock portion from being damaged at the base portion thereof due to the pressing of the needle base, even when the outer peripheral screw of the needle base is excessively screwed (for example, see Japanese Patent Application, First Publication No. Hei 10-272183).

A luer-lock type cylindrical tip of a syringe in which plural linear protrusions extending to the axial direction thereof are formed on the outer periphery of a cylindrical luer-lock portion at intervals in the peripheral direction is also known. With this luer-lock type cylindrical tip, even when a spiral protrusion on the outer periphery of the needle base of the injection needle is tightly screwed and fastened to the lock screw of the luer-lock portion, the luer-lock portion is not expanded outward, thereby preventing idle rotation of the spiral protrusions about the lock screw (for example, see Japanese Patent Application, First Publication No. 2000-271221).

Such luer-lock type cylindrical tips of a syringe can prevent the luer-lock portion from being damaged or deformed due to the excessive screwing or fastening of the needle base of the injection needle to the lock screw of the luer-lock portion. However, the shape of the screwing portion between the lock screw and the needle base of the injection needle is not considered at all. Accordingly, in the handling process after the injection needle is fitted to the cylindrical tip and until the syringe is finally used in the medical scene or the like, it is not possible to reliably prevent the injection needle from being loosened or pulled out relative to the cylindrical tip due to the vibration of the syringe or the like.

US 2,722,801, US 4,927,417 and JP 09-154947 disclose a luer-lock type cylindrical tip of a syringe according to the preamble of claim 1. FR 2 863 162 A1 discloses male connectors and female connectors which are used to produce liquid transmission connections, such as for enteral nutrition lines. The assembly of a male connector (RMI) or female connector (RFI), using a standardised female connector (RFN) or standardised male connector (RMN) respectively, is prevented because penetration of the tube of the male connector into the entry conduit of the female connector is impossible, or because this penetration is halted by the head of the female connector butting against the collar of the male connector.

The invention is contrived in view of the above-mentioned problems. An object of the invention is to provide a luer-lock type cylindrical tip of a syringe which can reliably prevent a injection needle from being loosened or pulled out relative to a cylindrical tip in the handling process after the injection needle is fitted to the cylindrical tip of the syringe in a manufacturing site and until the syringe is finally used in the medical scene or the like and which can allow the injection needle to be easily replaced with another one having different size at the time of use.

SUMMARY OF THE INVENTION

In order to accomplish the above-mentioned object, the invention has the following features.

According to a first aspect of the invention, there is provided a luer-lock type cylindrical tip of a syringe connecting to a distal end of a cylinder of the syringe, comprising a taper-shaped luer tip of which a center portion is fitted with a needle base of an injection needle and a cylindrical-shaped luer-lock portion which is disposed outside the luer tip with a gap therebetween and which has a lock screw formed therein. Here, the lock screw is formed as a trapezoid screw having a root diameter set to the range of 8.0 mm $\pm$ 0.1 mm, an inner diameter set to the range greater than or equal to 6.6 mm and smaller than 6.8 mm, and a pitch set to the range greater than 2.5 mm and smaller than or equal to 2.8 mm.

In the luer-lock type cylindrical tip according to the first aspect, it is preferable that the lock screw have a bottom thickness of a thread set to the range greater than 1.0 mm and smaller than or equal to 1.4 mm and a top thickness of the thread set to the range of 0.3 mm to 0.6 mm.

In the luer-lock type cylindrical tip according to the second aspect, it is preferable that the lock screw have a front inclination angle of the thread set to the range greater than 30° and smaller than or equal to 50° and a rear inclination angle of the thread set to the range of 25° to 50°.

According to the above-mentioned invention, the following excellent advantages can be obtained.

With the luer-lock type cylindrical tip according to the first aspect, it is possible to increase the height of the thread of the lock screw of the luer-lock portion. When the needle base of the injection needle is fitted to the luer tip to screw the screwing protrusion of the needle base to the lock screw, it is possible to enhance the area of a contact portion between the lock screw and the screwing protrusion, thereby increasing the frictional force acting on the contact portion. Accordingly, in the handling process after the injection needle is fitted to the cylindrical tip of the syringe in the manufacturing site and until the syringe is used in the medical scene or the like, it is possible to reliably prevent the injection needle from being loosened or pulled out relative to the cylindrical tip due to vibration or the like. In the lock screw, the inner diameter and pitch of the thread are slightly different from those prescribed in the ISO standard and other values can be set equal to those of the ISO standard can be employed. As a result, at the time of use, a user can easily replace the injection needle of the syringe with commercially available injection needles having dimensions corresponding to the ISO standard.

In addition, it is possible to increase the height of the thread of the lock screw of the luer-lock portion and to increase the thicknesses of the bottom and top of the thread. Accordingly, in the handling process, it is possible to reliably prevent the injection needles fitted to the cylindrical tip from being loosened or pulled out. Furthermore, it is possible to enhance the holding force of the lock screw for holding the injection needle in the cylindrical tip, thereby effectively enhancing the attachment strength of the injection needle to the cylindrical tip.

It is possible to set the height of the thread of the lock screw of the luer-lock portion, the thicknesses of the bottom and top of the thread, and the front and rear inclination angles of the thread to suitable ranges dissimilar but not different so much from the values of the ISO standard, thereby further effectively enhancing the attachment strength of the injection needle to the cylindrical tip. Furthermore, any injection needle can be fitted to the syringe for use without any inconvenience, as long as it has a commercially available needle base corresponding to the ISO standard.

BRIEF DESCRIPTION OF THE DRAWINGS

FIG. 1 is a longitudinal sectional view illustrating a part of a combined container-syringe having a luer-lock type cylindrical tip of a syringe according to an embodiment of the invention.

FIG. 2 is a longitudinal sectional view illustrating the luer-lock type cylindrical tip of a syringe according to the embodiment of the invention.

FIGS. 3A, 3B, and 3C are diagrams illustrating shapes of a needle base of an injection needle to be fitted to the luer-lock type cylindrical tip of a syringe according to the embodiment of the invention.

DETAILED DESCRIPTION OF THE INVENTION

Hereinafter, a luer-lock type cylindrical tip of a syringe according to an embodiment of the invention will be described with reference to the attached drawings.

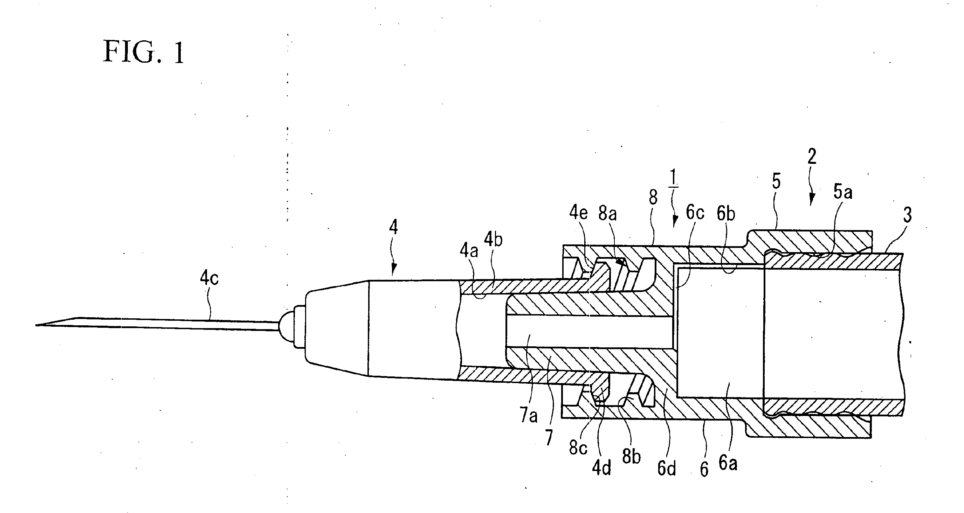

In FIG. 1, reference numeral 1 denotes a luer-lock type cylindrical tip of a syringe according to an embodiment of the invention, which is fitted to the outer periphery of a front end (left end in FIG. 1) of a cylinder 3 of a combined container-syringe (syringe) 2. Although not shown in detail, the combined container-syringe 2 further includes a finger grip disposed at the rear end of the cylinder 3, a front stopper and a rear stopper which are inserted into the cylinder 3 so as to reciprocate in the axis direction thereof and which seal a drug solution or drug product filled in the cylinder 3, a plunger rod which is inserted into the cylinder 3 from the rear side so as to connect the front end portion thereof to the rear stopper and to allow the rear stopper to reciprocate in the axis direction of the cylinder 3, an injection needle 4 fitted to the luer-lock type cylindrical tip 1, and a protector which covers the injection needle 4.

The luer-lock cylindrical tip 1 is made of, for example, a transparent or semitransparent synthetic resin. A bypass chamber portion 6 is disposed in front of a fitting hole 5a of a fitting portion 5 fitted to the cylinder 3. An inner space 6a having an inner diameter equal to or slightly larger than the inner diameter of the cylinder 3 is disposed in the bypass chamber portion 6. The front stopper is inserted into the inner space 6a. A cylindrical-shaped luer tip 7 is disposed in front of the bypass chamber portion 6. The luer tip 7 has a center portion communicating with the inner space 6a through a communication hole 7a and an outer periphery formed in a tapered cylindrical face. A luer-lock portion 8 along with the luer tip 7 is disposed in front of the bypass chamber portion 6. The luer-lock portion 8 has two right-handed lock screws 8a on the inner periphery with a gap from the outer periphery of the luer tip 7. The inner peripheral wall of the inner space 6a of the bypass chamber portion 6 is provided with a longitudinal groove 6b along the axis direction of the luer-lock type cylindrical tip 1. The bottom of the inner space 6a is provided with a lateral groove 6c for allowing the longitudinal groove 6b to communicate with the communication hole 7a. The basic structure of the luer-lock type cylindrical tip 1 is the same as generally known luer-lock type cylindrical tips as the conventional art.

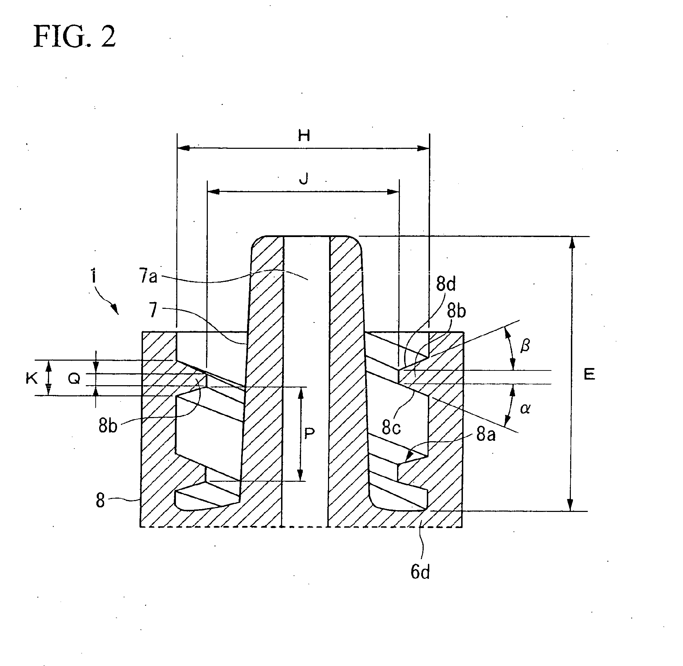

As shown in FIG. 2, the length from the outer surface of a bottom 6d of the bypass chamber 6 to the front end of the luer tip 7 is denoted by E, the root diameter of a thread 8b of the lock screw 8a is denoted by H, the inner diameter of the thread 8b is denoted by J, the thickness of the bottom of the thread 8b is denoted by K, the thickness of the top of the thread 8b is denoted by Q, the inclination angle of a front wall 8c of the thread 8b (front inclination angle) is denoted by α, the inclination angle of a rear wall 8d of the thread 8b (rear inclination angle) is denoted by β, and the pitch of the thread 8b is denoted by P. In the invention, the dimensions of the luer tip 7 and the luer-lock portion 8 are set to the values shown in Table 1. In Table 1, the dimensions E, H, J, K, Q, α, β, and P are shown for comparison with the dimensions prescribed in the ISO standard.

Table 1

| Dimensions of Luer Tip and Luer-Lock portion (unit: mm) | | | |
|---|---|---|---|
| Symbol | The invention | Symbol | ISO594-2 Standard |
| E | 7.5 in minimum | E | 7.5 in minimum |
| H | 8.0±0.1 | H | 8.0±0.1 |
| J | 6.6 ~ 6.8 (not equal thereto) | J | 7.0±0.2 |
| K | 1.0 (not equal thereto) ~ 1.4 | K | 1.0 in maximum |
| Q | 0.3 ~ 0.6 | Q | 0.3 in minimum |
| α | 30˚ ~ 50˚ | α | $25° \, {}^{+5°}_{0°}$ |
| β | 25˚ ~ 50˚ | β | 25˚ in minimum |
| P | 2.5 (not equal thereto) ~ 2.8 | P | 2.5 |

The injection needle 4 is constructed by attaching a needle tube 4c to the front end of the needle base 4b having a tapered hole (inner hole) 4a fitted to the outer periphery of the luer tip 7 so as to allow a tube hole of the needle tube 4c to communicate with the tapered hole 4a. Screwing protrusions 4d screwed to the lock screw 8a of the luer-lock portion 8 are disposed on the outer periphery of the base of the needle base 4b of the injection needle 4.

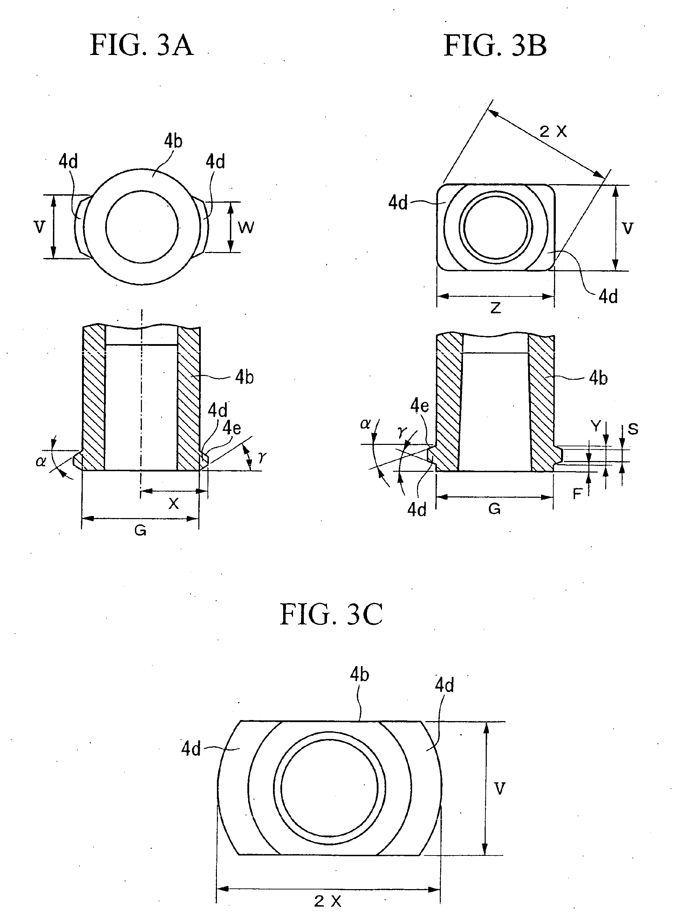

The needle base 4b has one of three kinds of dimensions and shapes prescribed in the ISO standard shown in FIGS. 3A to 3C. The needle base 4b shown in FIG. 3A is made of a synthetic resin having semi-rigidity. The screwing protrusions 4d are disposed oppositely in the diameter direction of the needle base 4b at the end of the base portion of the needle base 4b having an outer diameter G. The screwing protrusion 4d has an arc-like top shape having a radius X, a base width V, and a tip width W and a trapezoid-like sectional shape having a rear inclination angle γ and a front inclination angle α. The needle base 4b shown in FIG. 3B is made of rigid metal. The screwing protrusions 4d are disposed oppositely in the diameter direction of the needle base 4b at a position moved inside by length F from the end of the base portion of the needle base 4b having an outer diameter G. The screwing protrusion 4d has a rectangular top shape having a width V, a length Z, and a diagonal length 2X and a trapezoid-like sectional shape having a bottom thickness Y, a top thickness S, a rear inclination angle γ, and a front inclination angle α. The needle base 4b shown in FIG. 3C is made of rigid metal. The screwing protrusion 4d has an arc-like top shape having a width V and a diameter 2X and a sectional shape equal to that of the screwing protrusions 4d shown in FIG. 3B.

The needle bases 4b having the screwing protrusions 4d of all dimensions and shapes can be smoothly and reliably fitted to the lock screw 8a of the luer-lock portion 8. However, when the dimensions J, K, Q, α, β, and P of the lock screw 8a are set to values departing from the allowable ranges, the commercially available needle bases 4b corresponding to the ISO standard may not be accurately and precisely fitted to the lock screw 8a.

In the luer-lock type cylindrical tip 1 of the combined container-syringe 2 having the above-mentioned configuration, an injection needle 4 having one of the needle bases 4b shown in FIGS. 3A, 3B, and 3C is selected. The needle base 4b of the selected injection needle 4 is inserted into the luer-lock portion 8. That is, the luer tip 7 is inserted into the tapered hole 4a of the needle base 4b. When the screwing protrusions 4d of the needle base 4b come in contact with the lock screw 8a, the needle base 4b is rotated to screw the screwing protrusions 4d to the lock screw 8a.

Accordingly, the tapered hole 4a of the needle base 4b is fitted to the luer tip 7 and a rear wall 4e of the screwing protrusion 4d engages with and comes in contact with the front wall 8c of the thread 8b of the lock screw 8a. Accordingly, the needle base 4b is fastened to the lock screw 8a and the injection needle 4 is firmly fitted to the luer-lock portion 8.

Here, as shown in Table 1, the bottom thickness K of the thread 8b of the lock screw 8a is set to the range greater than 1.0 mm and smaller than or equal to 1.4 mm, the top thickness Q of the thread 8b is set to the range of 0.3 mm to 0.6 mm, the front inclination angle $\alpha$ of the thread 8b is set to the range greater than 30˚ and smaller than or equal to 50˚, and the rear inclination angle $\beta$ of the thread 8b is set to the range of 25˚ to 50˚. Accordingly, the bottom thickness of the thread 8b can be set substantially larger than the dimension prescribed in the ISO standard, thereby enhancing the strength of the thread 8b.

Therefore, even when the needle base 4b is screwed to the lock screw 8a with a large fastening force so as to prevent the loosening of the injection needle 4 relative to the luer-lock portion 8, the thread 8b of the lock screw 8a is neither broken nor damaged. In addition, it is possible to greatly enhance the strength resisting to the force pulling the injection needle 4 out of the luer-lock portion 8 (pullout strength of the injection needle 4).

The inner diameter J of the thread 8b of the lock screw 8a can be set smaller than the dimension prescribed in the ISO standard, thereby greatly enhancing the area of the contact portion between the front wall 8c of the thread 8b and the rear wall 4e of the screwing protrusion 4d of the needle base 4b. Accordingly, it is possible to enhance the frictional force acting on the contact portion by means of the fastening force of the needle base 4b relative to the lock screw 8a. In the combined container-syringe 2 with the injection needle 4 fitted to the luer-lock type cylindrical tip 1, even when the force for loosening the screw coupling acts on the screwing portion between the lock screw 8a and the screwing protrusion 4d of the needle base 4b due to vibrations or the like in the handling process from the assembly thereof to use in the medical scene or the like, the force resisting the loosening acts sufficiently and thus the screwing portion is not loosened or pulled out. Accordingly, it is possible to greatly enhance the anti-loosening strength of the injection needle 4.

Next, the attachment strength of the attachment portion due to the screwing between the lock screw 8a of the luer-lock portion 8 and the needle base 4b of the injection needle 4 made of a synthetic resin material (the attachment strength of the injection needle 4 to the luer-lock type cylindrical tip 1) is compared with that of the case using the luer-lock portion made of a synthetic resin material having the dimensions prescribed in the ISO standard and the comparison test result is described.

The dimensions of the luer-lock portion 8 according to the invention used in the comparison test were H=8.0 mm, J=6.7 mm, K=1.2 mm, Q=0.4 mm, $\alpha$=45˚, $\beta$=30˚, and P=2.6 mm, which is within the range shown in Table 1. The dimensions of the luer-lock portion corresponding to the ISO standard were H=8.0 mm, J=7.0 mm, K=1.0 mm, Q=0.3 mm, $\alpha$=30˚, $\beta$=25˚, and P=2.5 mm, which is within the range shown in Table 1.

The attachment strengths of the injection needle 4 to the luer-lock type cylindrical tip 1 were measured as follows.

(1) The injection needle 4 was screwed to the luer-lock portion 8, the luer-lock portion 8 was fixed to a fixing jig, and the injection needle 4 was drawn by a chuck of a force gauge in the axial direction where it is separated from the luer-lock portion 8 with a constant weight. The force when the injection needle 4 was separated from the luer-lock portion 8 was read from the scale of the force gauge and was used as the pullout strength of the injection needle 4. (2) The luer-lock portion 8 to which the injection needle 4 had been screwed was fixed to a fixing jig and a rotation torque was applied to the injection needle 4 by the use of a chuck of a torque gauge in the direction where the injection needle was loosened and which was opposite to the assembly direction. The rotation torque when the injection needle 4 started to be loosened was read from the scale of the torque gauge and used as the anti-loosening strength of the syringe 4.

The comparison test was performed on 10 samples each from two production lots each for the luer-lock portion 8 according to the invention and the luer-lock portion corresponding to the ISO standard. The average value, the maximum value, the minimum value, and the standard deviation value were calculated based on the measured values. The results of the comparison test are listed in Tables 2 and 3.

Table 2

| Pullout Strength of Injection Needle (unit: N) | | | | |
|---|---|---|---|---|
| Sample No. | The invention | | ISO594-2 Standard | |
| | Lot No. S1 | Lot No. S2 | Lot No. S3 | Lot No. S4 |
| 1 | 80.0 | 91.0 | 38.6 | 31.2 |
| 2 | 82.2 | 75.0 | 42.3 | 30.4 |

(continued)

| Pullout Strength of Injection Needle (unit: N) | | | | |
|---|---|---|---|---|
| Sample No. | The invention | | ISO594-2 Standard | |
| | Lot No. S1 | Lot No. S2 | Lot No. S3 | Lot No. S4 |
| 3 | 68.4 | 68.2 | 41.1 | 26.6 |
| 4 | 91.9 | 77.2 | 47.8 | 44.7 |
| 5 | 79.0 | 75.2 | 43.7 | 36.7 |
| 6 | 85.5 | 83.0 | 36.4 | 45.2 |
| 7 | 84.0 | 72.1 | 33.7 | 34.7 |
| 8 | 68.8 | 90.2 | 37.9 | 46.5 |
| 9 | 72.9 | 75.6 | 38.4 | 43.2 |
| 10 | 68.2 | 79.0 | 40.5 | 34.9 |
| Average | 78.09 | 78.65 | 40.04 | 37.41 |
| Maximum | 91.9 | 91.0 | 47.8 | 46.5 |
| Minimum | 68.2 | 68.2 | 33.7 | 26.6 |
| Standard deviation | 8.21 | 7.41 | 3.98 | 7.06 |

Table 3

| Anti-loosening Strength of Injection Needle (unit: N.m) | | | | |
|---|---|---|---|---|
| Sample No. | The invention | | ISO594-2 Standard | |
| | Lot No. S1 | Lot No. S2 | Lot No. S3 | Lot No. S4 |
| 1 | 10.8 | 10.2 | 7.7 | 7.1 |
| 2 | 10.2 | 11.1 | 6.8 | 7.3 |
| 3 | 10.7 | 10.5 | 6.7 | 6.9 |
| 4 | 11.2 | 14.3 | 7.6 | 6.1 |
| 5 | 11.5 | 9.9 | 7.6 | 6.9 |
| 6 | 11.3 | 11.1 | 7.4 | 7.0 |
| 7 | 12.4 | 10.9 | 6.7 | 5.6 |
| 8 | 9.9 | 9.9 | 6.7 | 6.2 |
| 9 | 11.9 | 10.5 | 6.8 | 4.4 |
| 10 | 10.7 | 10.7 | 7.4 | 5.8 |
| Average | 11.06 | 10.91 | 7.14 | 6.33 |
| Maximum | 12.4 | 14.3 | 7.7 | 7.3 |
| Minimum | 9.9 | 9.9 | 6.7 | 4.4 |
| Standard deviation | 0.76 | 1.27 | 0.43 | 0.90 |

As can be seen from the values of the comparison test results shown in Table 2, by setting the dimensions of the lock screw 8a of the luer-lock portion 8 to predetermined values, the pullout strength of the injection needle 4 in the case when the luer-lock portion 8 according to the invention was used could be further enhanced by 1.96 to 2.09 times compared to that of the luer-lock portion corresponding to the ISO standard. As can be seen from the values of the comparison test results shown in Table 3, despite screwing the needle base 4b being made of a synthetic resin which was more slidable than a metal to the lock screw 8a, the anti-loosening strength of the injection needle 4 in the case when the luer-lock portion 8 according to the invention was used could be further enhanced by 55% to 72% compared to that of the luer-lock portion corresponding to the ISO standard. In the comparison test results shown in Tables 2 and 3, the dimensions of the lock screw 8a of the luer-lock portion 8 were set to the specific values in the ranges shown in Table 1. However, even when the dimensions were set to other values in the ranges shown in Table 1, the same test results could be obtained.

As described above, the luer-lock type cylindrical tip 1 of the combined container-syringe 2 according to the embodiment of the invention is a luer-lock type cylindrical tip connecting to the distal end of the cylinder 3 of the combined container-syringe 2, comprising the cylindrical-shaped luer tip 7 of which the center portion is fitted with the needle base 4b of the injection needle 4 and the cylindrical-shaped luer-lock portion 8 which is disposed outside the luer tip 7 with a gap therebetween and which has the lock screw 8a formed therein. The lock screw 8a is formed as a trapezoid-like screw having a root diameter H set to the range of 8.0 mm $\pm$ 0.1 mm, an inner diameter J set to the range greater than or equal to 6.6 mm and smaller than 6.8 mm, a bottom thickness K of the thread 8b set to the range greater than 1.0 mm and smaller than or equal to 1.4 mm, a top thickness Q of the thread 8b set to the range of 0.3 mm to 0.6 mm, a front inclination angle $\alpha$ of the front wall 8c of the thread 8b set to the range greater than 30˚ and smaller than or equal to 50˚, a rear inclination angle $\beta$ of the rear wall 8d of the thread 8b set to the range of 25˚ to 50˚, and a pitch P set to the range greater than 2.5 mm and smaller than or equal to 2.8 mm.

With the luer-lock type cylindrical tip 1 of a syringe according to the embodiment, it is possible to enhance the height of the thread 8b of the lock screw 8a of the luer-lock portion 8. Accordingly, when the needle base 4b of the injection needle 4 is fitted to the luer tip 7 to screw the screwing protrusion 4d of the needle base 4b to the lock screw 8a, it is possible to increase the area of the contact portion between the lock screw 8a and the screwing protrusion 4d, thereby enhancing the frictional force acting on the contact portion. Therefore, in the handling process until the injection needle 4 attached to the luer-lock type cylindrical tip 1 of the combined container-syringe 2 in the manufacturing site is used in the medical scene or the like, it is possible to reliably prevent the injection needle 4 from being loosened or pulled out relative to the cylindrical tip 1 due to vibrations or the like, thereby effectively enhancing the anti-loosening strength of the injection needle 4.

It is possible to increase the bottom and top thicknesses of the thread 8b of the lock screw 8a of the luer-lock portion 8. Accordingly, even when a large force for pulling the injection needle 4 out of the luer tip 7 acts on the injection needle 4 of which the needle base 4b is fitted to the luer tip 7 to screw the screwing protrusion 4d of the needle base 4b to the lock screw 8a, it is possible for the lock screw 8a to sufficiently resist the force and to hold the injection needle 4 in the luer-lock type cylindrical tip 1 without breaking or damaging, thereby effectively enhancing the pullout strength of the injection needle 4.

In the lock screw 8a, the inner diameter J of the thread 8b, thicknesses K and Q of the bottom and top of the thread 8b, and pitch P of the thread 8b are slightly different from those of the ISO standard and other values can be set equal to those of the ISO standard. As a result, at the time of use, a user can easily replace the injection needles 4 of the combined container-syringe 2 with commercially available injection needles having dimensions corresponding to the ISO standard.

In this way, with the luer-lock cylindrical tip 1 according to the above-mentioned embodiment, it is possible to set the height of the thread 8b of the lock screw 8a of the luer-lock portion 8, the top and bottom thicknesses K and Q of the thread 8b, the front and rear inclination angels $\alpha$ and $\beta$ of the thread 8b, and the pitch P to suitable ranges dissimilar but not different so much from the dimensions prescribed in the ISO standard. It is also possible to both enhance the anti-loosening strength of the injection needle 4 and the pullout strength of the injection needle 4, thereby effectively enhancing the attachment strength of the injection needle 4 to the luer-lock type cylindrical tip 1. Any injection needle 4 can be fitted for use without any inconvenience, as long as it has a commercially available needle base 4b corresponding to the ISO standard.

In the luer-lock type cylindrical tip 1 of a syringe according to the above-mentioned embodiment, the dimensions J, K, Q, $\alpha$, $\beta$, and P of the lock screw 8a of the luer-lock portion 8 are set to the ranges shown in Table 1. By setting the dimensions of the lock screw 8a to be different from the dimensions prescribed in the ISO standard, it is possible to effectively enhance both the pullout strength of the injection needle 4 and the anti-loosening strength of the injection needle 4. However, the invention is not limited to the embodiment. The anti-loosening strength of the injection needle 4 may be primarily increased to enhance the attachment strength of the injection needle 4 to the luer-lock type cylindrical tip 1 by setting the dimensions J and P of the lock screw 8a to the ranges shown in Table 1 and setting the other dimensions to the values prescribed in the ISO standard. By setting the dimensions K, Q, and P of the lock screw 8a to the ranges shown in Table 1 and setting the other dimensions to the values prescribed in the ISO standard, the pullout strength of the injection needle 4 may be primarily increased to enhance the attachment strength of the injection needle 4 to the luer-lock type cylindrical tip 1.

In the luer-lock type cylindrical tip 1 of a syringe according to the above-mentioned embodiment, the luer-lock type cylindrical tip 1 that is fitted to the front end portion of the cylinder 3 is illustrated. However, the invention is not limited to the embodiment, but may be applied to an example where the luer-lock type cylindrical tip is integrally incorporated into the cylinder 3. Although the invention has been applied to the cylindrical tip of the combined container-syringe in which a drug solution has been filled in advance in the cylinder 3, the invention is not limited to the example, but may be applied to cylindrical tips of other syringes.

While preferred embodiments of the invention have been described and illustrated above, it should be understood that these are exemplary of the invention and are not to be considered as limiting. Additions, omissions, substitutions, and other modifications can be made without departing from the scope of the present invention. Accordingly, the invention is not to be considered as being limited by the foregoing description, and is only limited by the scope of the appended claims.

**Claims**

1. A luer-lock type cylindrical tip (1) of a syringe (2) connecting to a distal end of a cylinder (3) of the syringe, comprising:

     a taper-shaped luer tip (7) of which a center portion is fitted with a needle base (4b) of an injection needle (4); and
     a cylindrical-shaped luer-lock portion (8) which is disposed outside the luer tip with a gap therebetween and which has a lock screw (8a) formed therein, **characterized in that**:

          the lock screw is formed as a trapezoid screw having a root diameter set to the range of 8.0 mm $\pm$ 0.1 mm, an inner diameter set to the range greater than or equal to 6.6 mm and smaller than 6.8 mm, and a pitch set to the range greater than 2.5 mm and smaller than or equal to 2.8 mm.

2. The luer-lock type cylindrical tip according to claim 1, wherein the lock screw has a bottom thickness of a thread set to a range greater than 1.0 mm and smaller than or equal to 1.4 mm and a top thickness of the thread set to a range of 0.3 mm to 0.6 mm.

3. The luer-lock type cylindrical tip according to claim 2, wherein the lock screw has a front inclination angle of the thread set to a range greater than 30˚ and smaller than or equal to 50˚ and a rear inclination angle of the thread set to a range of 25˚ to 50˚

**Patentansprüche**

1. Zylindrische Luer-Lock-Spitze (1) einer Spritze (2), welche an ein Distalende eines Zylinders (3) der Spritze koppelt, umfassend:

     eine kegelförmige Luer-Spitze (7), von der ein Mittelteil mit einem Nadelgehäuseunterteil (4b) einer Injektionsnadel (4) ausgerüstet ist; und
     einen zylinderförmigen Luer-Lock-Teil (8), welcher außerhalb der Luer-Spitze mit einer dazwischen liegenden Lücke angeordnet ist und welcher eine darin ausgebildete Schraubensicherung (8a) aufweist, **dadurch gekennzeichnet,
     dass**
     die Schraubensicherung als eine trapezförmige Schraube ausgebildet ist, welche einen Grunddurchmesser aufweist, der im Bereich von 8,0 mm $\pm$ 0,1 mm eingestellt ist, einen inneren Durchmesser, der im Bereich größer oder gleich 6,6 mm und kleiner als 6,8 mm eingestellt ist, und eine Ganghöhe, welche für den Bereich größer als 2,5 mm und kleiner oder gleich 2,8 mm eingestellt ist.

2. Zylindrische Luer-Lock-Spitze nach Anspruch 1, wobei die Schraubensicherung eine zur Unterseite gehörige Dicke eines Gewindes aufweist, welche für einen Bereich größer als 1,0 mm und kleiner oder gleich 1,4 mm eingestellt ist, und eine zur Oberseite gehörige Dicke des Gewindes, welche für einen Bereich 0,3 mm bis 0,6 mm eingestellt ist.

3. Zylindrische Luer-Lock-Spitze nach Anspruch 2, wobei die Schraubensicherung einen vorderen Neigungswinkel des Gewindes aufweist, welcher für einen Bereich größer als 30˚ und kleiner oder gleich 50˚ eingestellt ist, und einen hinteren Neigungswinkel des Gewindes, welcher für einen Bereich von 25˚ bis 50˚ eingestellt ist.

**Revendications**

1. Embout (1) cylindrique de type Luer-Lock d'une seringue (2) se raccordant à une extrémité distale d'un cylindre (3) de la seringue, comprenant :

   un embout Luer (7) en forme conique dont une partie centrale est munie d'une embase d'aiguille (4b) d'une aiguille d'injection (4) ; et
   une partie Luer-Lock (8) de forme cylindrique qui est disposée à l'extérieur de l'embout Luer avec un espace entre eux et qui a une vis d'obturation (8a) formée à l'intérieur, **caractérisé en ce que** :

   la vis d'obturation est formée sous forme de vis trapézoïdale ayant un diamètre de fond de filet réglé dans la plage allant de 8,0 mm $\pm$ 0,1 mm, un diamètre intérieur réglé dans une plage de façon à être supérieur ou égal à 6,6 mm et inférieur à 6,8 mm, et un pas réglé dans une plage de façon à être supérieur à 2,5 mm et inférieur ou égal à 2,8 mm.

2. Embout cylindrique de type Luer-Lock selon la revendication 1, dans lequel la vis d'obturation présente une épaisseur inférieure d'un filetage réglée dans une plage de façon à être supérieure à 1,0 mm et inférieure ou égale à 1,4 mm et une épaisseur supérieure du filetage réglée dans une plage allant de 0,3 mm à 0,6 mm.

3. Embout cylindrique de type Luer-Lock selon la revendication 2, dans lequel la vis d'obturation présente un angle d'inclinaison avant du filetage réglé dans une plage de façon à être supérieur à 30˚ et inférieur ou égal à 50˚ et un angle d'inclinaison arrière du filetage réglé dans une plage allant de 25˚ à 50˚.

FIG. 1

EP 1 923 086 B1

FIG. 2

FIG. 3A

FIG. 3B

FIG. 3C

**EP 1 923 086 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10272183 A
- JP 2000271221 A
- US 2722801 A
- US 4927417 A
- JP 9154947 A
- FR 2863162 A1